# EUROPÄISCHE PATENTSCHRIFT

(11) **EP 2 145 586 B1**
(45) Veröffentlichungstag und Bekanntmachung des Hinweises auf die Patenterteilung: **31.12.2014**
(21) Anmeldenummer: 08160536.2
(22) Anmeldetag: 16.07.2008
(51) Int. Cl.: A61B 19/00, A61B 19/02, B25J 9/00

(54) **Adapter zur Fixierung eines Medizingerätes**
Adapter for mounting a medical device
Adaptateur destiné à la fixation d'un appareil médical

(43) Veröffentlichungstag der Anmeldung: 20.01.2010
(73) Patentinhaber: Brainlab AG, 85622 Feldkirchen (DE)
(72) Erfinder: Blechinger, Steffi, 85591, Vaterstetten (DE); Blum, Stefanie, 80805, München (DE); Roßner, Holger, 85622, Feldkirchen (DE); Wohlgemuth, Richard, 83646, Bad Tölz (DE)
(74) Vertreter: Schwabe - Sandmair - Marx

(56) Entgegenhaltungen:
- EP-A- 1 190 680
- WO-A-02/065933
- WO-A-02/085187
- US-A- 3 221 743

## Beschreibung

Die vorliegende Erfindung betrifft einen Adapter zur Fixierung eines Medizingerätes an einer oder zwei Supportstrukturen.

In medizinischen Anwendungen werden häufig Geräte verwendet, die schwer sind oder äußerst präzise geführt werden müssen. Es ist deshalb notwendig, Halterungen für derartige Geräte bereitzustellen. So offenbaren die Patente US 5,657,429 und US 5,553,198 jeweils ein Robotersystem, bei dem ein Roboterarm mittels eines Adapters auf einem Wagen oder an einem Operationstisch befestigbar ist. Der Roboterarm ist fest mit dem Adapter verbunden und über einen Mechanismus entweder mit dem Operationstisch oder dem Wagen verbunden.

Das Dokument WO 02/085187 A2, aus dem der Anspruch 1 abgeleitet wird, offenbart einen radioluzenten, halo-artigen Retraktor mit einem radioluzenten Retraktorring, der auf oberen Enden von radioluzenten Halteklammern montiert ist. Die Halteklammem sind jeweils an ihren unteren Enden an radioluzenten Haltebügeln befestigt. An dem Retraktorring können Retraktorarme befestigt werden.

Es ist die Aufgabe der vorliegenden Erfindung, einen einfacher aufgebauten und flexibler einsetzbaren Adapter zur Fixierung eines Medizingerätes an einer Supportstruktur bereitzustellen.

Gelöst wird diese Aufgabe durch einen Adapter nach Patentanspruch 1. Der Patentanspruch 12 betrifft ein medizinisches System mit einem solchen Adapter. Vorteilhafte Ausgestaltungsformen sind den abhängigen Patentansprüchen zu entnehmen.

Ein Adapter zur Fixierung eines Medizingerätes an einer oder zwei Supportstrukturen ist erfindungsgemäß dreiteilig aus einem Trageteil und zwei Supportteilen aufgebaut, wobei das Trageteil mit dem Medizingerät verbindbar ist, das erste Supportteil mit einer ersten Supportstruktur verbindbar ist und das zweite Supportteil mit einer zweiten Supportstruktur verbindbar ist. Die erste Supportstruktur ist beispielsweise ein Operationstisch bzw. eine an einem Operationstisch befestigte Schiene. Das zweite Supportteil ist beispielsweise ein horizontal verfahrbares, vertikal höhenverstellbares Stativ, dessen Räder optional einfahrbar sind, so dass der Fuß des Stativs fest auf dem Boden steht. Bei dem Medizingerät handelt es sich beispielsweise um einen Roboterarm.

Der Adapter kann erfindungsgemäß zumindest drei Verbindungszustände einnehmen, wobei im ersten Zustand das Trageteil spielfrei nur mit dem ersten Supportteil verbunden ist, im zweiten Zustand das Trageteil spielfrei nur mit dem zweiten Supportteil verbunden ist und im dritten Zustand das Trageteil spielfrei mit dem ersten Supportteil und dem zweiten Supportteil verbunden ist. Der dritte Zustand bietet den Vorteil, dass die erste Supportstruktur, beispielsweise ein Operationstisch, über den Adapter von der zweiten Supportstruktur zusätzlich getragen werden kann und dadurch zusätzlich stabilisiert wird, insbesondere wenn das Medizingerät ein hohes Gewicht aufweist und dadurch ein Moment auf die erste Supportstruktur ausübt. Bei einer speziellen Ausgestaltung einer spielfreien Verbindung sind die miteinander verbundenen Teile gegeneinander fixiert, sodass keine rotatorische oder translatorische Relativbewegung zwischen den Teilen möglich ist.

In einer bevorzugten Ausführungsform der Erfindung sind das Trageteil und das erste Supportteil in genau einer Position spielfrei miteinander verbindbar. Diese relative Position wird auch als Endposition zwischen den beiden Teilen bezeichnet. Das bedeutet, dass die relative Position zwischen dem Trageteil und dem ersten Supportteil bei einer Verbindung der beiden Teile exakt bekannt ist. Damit ist auch die relative Position des Medizingerätes zur ersten Supportstruktur bekannt.

In vorteilhafter Weise sind das Trageteil und das erste Supportteil zur gegenseitigen Verbindung miteinander insbesondere vertikal verschiebbar ausgebildet. Dazu weist das Trageteil z. B. ein Verschiebeteil, insbesondere eine Platte, auf, das dazu ausgebildet ist an einem Element des ersten Supportteils verschiebbar angebracht zu werden, also z. B. in taschenartige Schienen am ersten Supportteil eingeführt oder an einer Stange des Supportteils verschiebbar angebracht zu werden. Das Verschiebeteil des Trageteils wird in die Schienen am ersten Supportteil eingeschoben, um eine Verbindung zwischen den beiden Teilen herzustellen. Bevorzugt weisen die Schienen einen Endanschlag für das Verschiebeteil auf, falls sich das Verschiebeteil und somit das Trageteil in seiner Endposition befindet, in der das Trageteil und das erste Supportteil fest und spielfrei miteinander verbunden werden. Dabei ist das erste Supportteil bevorzugt derart an der ersten Supportstruktur angeordnet, dass die taschenartigen Schienen in vertikaler Richtung verlaufen. Die auf das Trageteil wirkende Schwerkraft drückt dann das Verschiebeteil in Richtung des Bodens der von den Schienen gebildeten Tasche.

Weiterhin bevorzugt weisen die taschenartigen Schienen am ersten Supportteil an ihrem Ende mindestens eine schräge Anlagefläche auf. Da zwischen den taschenartigen Schienen und dem Verschiebeteil ein geringes Spiel vorgesehen sein sollte, um das Verschiebeteil möglichst reibungsfrei in die Schienen einzuführen, sorgt die schräge Anlagefläche in der Endposition für eine definierte Anlage des Verschiebeteils in den taschenartigen Schienen und damit am ersten Supportteil. Die Fläche ist beispielsweise keilförmig angeschrägt. Bei der bevorzugten vertikalen Ausrichtung der Schienen erzeugt die Schwerkraft eine Klemmkraft zwischen den Schienen und dem Verschiebeteil, die zu einer spielfreien Verbindung führt.

In einer Ausgestaltungsform der Erfindung ist am ersten Supportteil eine Fixierungseinrichtung zur Fixierung des Trageteils gegenüber dem ersten Supportteil angeordnet. Durch die Fixierungseinrichtung wird beispielsweise das Verschiebeteil in den taschenartigen Schienen festgeklemmt. Die Fixierung durch die Fixierungseinrichtung erfolgt zusätzlich oder alternativ zu der Fixierung durch die Schwerkraft.

Der Adapter weist bevorzugt eine Zentrierhilfe zur Vereinfachung der Verbindung zwischen dem Trageteil und dem ersten Supportteil auf. Die Zentrierhilfe besteht beispielsweise aus einer oder mehreren schrägen Flächen, die das Trageteil bei der Annäherung an das erste Supportteil und/oder beim Einschieben der Platte am Trageteil in die taschenartigen Schienen am ersten Supportteil zentrieren.

Bevorzugt weist der Adapter einen Verschluss zur Sicherung des Trageteils gegenüber dem ersten Supportteil auf. Bei dem Verschluss handelt es sich beispielsweise um einen Schubspanner, einen Exzenterhebel, einen Exzenterhebel mit Feder oder einen abgeschrägten Bolzen. Der Verschluss verhindert im geschlossenen Zustand ein Trennen von Trageteil und erstem Supportteil.
ein auf einem Stativ angebrachtes zweites Supportteil in mehreren Höhen des Stativs, beispielsweise wenn das Stativ in der Höhe verstellt oder auf den Boden abgesenkt wurde, spielfrei mit dem Trageteil verbunden werden. Dies ist beispielsweise dadurch möglich, dass das zweite Supportteil ein zylindrisches Grundteil aufweist, das in einen hülsenartigen Bereich des Trageteils einführbar ist. Das zweite Supportteil und das Trageteil sind dann in mehreren, axial verschobenen Positionen des zylindrischen Grundteils gegenüber dem Trageteil spielfrei miteinander verbindbar. Der hülsenartige Bereich und das zylindrisches Grundteil bilden so eine klemmbare Verschiebehülse. Der Begriff "axial" bezieht sich dabei auf die Mittelachse des zylindrischen Grundteils, die mit der Mittelachse des hülsenartigen Bereichs des Trageteils identisch ist. Bevorzugt weist diese Achse in Richtung der Schwerkraft, liegt also in der Vertikalen.

Der hülsenartige Bereich des Trageteils besteht beispielsweise aus zwei Halbschalen, die mittels eines Scharniers miteinander verbunden sind und durch einen Verschlussmechanismus verschließbar sind. Der Verschlussmechanismus basiert beispielsweise auf einem Klemmhebel oder einem Exzenterhebel. Durch das Verschließen des hülsenartigen Bereichs ergibt sich ein zylindrischer Pressverband zwischen dem Trageteil und dem zylindrischen Grundteil des zweiten Supportteils. Die relative Position des Supportteils und des Trageteils wird somit spielfrei aufgrund von Reibung gehalten.
Eine andere Möglichkeit zur spielfreien Verbindung von Trageteil und zweitem Supportteil ist ein Klemmmechanismus, bei dem ein Bereich eines der beiden Teile gespreizt werden kann und auf einen inneren oder äußeren Bereich des jeweiligen anderen Teils drückt.

Bevorzugt weist der Adapter eine Zentrierhilfe zur Vereinfachung der Verbindung zwischen dem Trageteil und dem zweiten Supportteil auf. Dabei handelt es sich beispielsweise um eine Fase an der Außenseite des in den hülsenartigen Bereich des Trägerteils einführbaren zylindrischen Grundteils des zweiten Supportteils. Alternativ oder zusätzlich weist der hülsenartige Bereich des Trägerteils an der Seite, aus der das zylindrische Grundteil des zweiten Supportteils in den hülsenartigen Bereich des Trageteils einführbar ist, eine trichterförmige Anfasung auf.

Bevorzugt ist der Adapter so ausgestaltet, dass die Verbindung zwischen dem Trageteil und dem zweiten Supportteil nur trennbar ist, wenn eine Verbindung zwischen dem Trageteil und dem ersten Supportteil besteht. Dies geschieht beispielsweise mittels eines Stifts, der eine Relativbewegung zwischen dem Trageteil und dem zweiten Supportteil verhindert. Die Blockierung durch den Stift wird beispielsweise dadurch gelöst, dass der Stift über einen Hebelmechanismus aus dem Eingriff gebracht wird, sobald das Trageteil seine Endposition gegenüber dem ersten Supportteil erreicht hat. Der Hebelmechanismus ist beispielsweise am Trageteil angeordnet und wird vom ersten Supportteil betätigt. Der Hebelmechanismus ist bevorzugt über eine Feder vorgespannt, so dass der Stift automatisch in Eingriff gebracht wird, wenn der Hebelmechanismus nicht betätigt wird.

Bevorzugt ist mindestens eines der drei Teile des Adapters, insbesondere das erste Supportteil, sterilisierbar. Dies betrifft bevorzugt alle Komponenten, aus denen das sterilisierbare Teil besteht. Die drei Teile bzw. ihre Komponenten sind beispielsweise aus Aluminium oder einer Aluminiumlegierung wie AlMg4,5Mn gefertigt.

Die Erfindung betrifft weiterhin ein medizinisches System mit einem Medizingerät, einer ersten Supportstruktur, einer zweiten Supportstruktur und einem Adapter, wie er vorstehend beschrieben wurde. Das erste Supportteil des Adapters ist mit der ersten Supportstruktur verbunden, das zweite Supportteil des Adapters ist mit der zweiten Supportstruktur verbunden und das Trageteil des Adapters ist mit dem Medizingerät verbunden. Das Medizingerät kann beispielsweise ein Roboterarm, ein Mikroskop, ein Ultraschallgerät, ein Röntgengerät, eine Operationslampe oder auch eine 3D-Kamera für die bildgestützte Navigation sein. Die relative Lage der beiden Supportstrukturen und des Medizingeräts zueinander wird durch die relative Lage der Teile des Adapters zueinander bestimmt. Die erste Supportstruktur ist beispielsweise ein Operationstisch, die zweite Supportstruktur ist beispielsweise ein Stativ. Der Operationstisch weist beispielsweise eine Schiene auf, an der das erste Supportteil befestigt wird. Dies kann durch eine Verschraubung erfolgen, die entweder starr ausgebildet ist oder einen Klappmechanismus aufweist. Alternativ kann ein Exzenterklemmhebel verwendet werden.

Das medizinische System weist bevorzugt mindestens einen Sensor zur Erfassung des Zustands des Adapters auf. Der Zustand des Adapters setzt sich aus verschiedenen Unterzuständen zusammen. Mögliche Unterzustände sind beispielsweise eine Berührung des Trageteils und des ersten Supportteils, eine Endposition des Trageteils in Bezug auf das erste Supportteil, eine Vorschubbewegung des Trageteils gegenüber dem ersten Supportteil, eine fixierte Verbindung zwischen dem Trageteil und dem ersten Supportteil, eine fixierte Verbindung zwischen dem Trageteil und dem zweiten Supportteil, eine Berührung des Trageteils und des zweiten Supportteils oder ein Vorschub des zweiten Supportteils gegenüber dem Trageteil.

Bevorzugt werden ein oder mehrere der vorgenannten Unterzustände detektiert, wobei beispielsweise für jeden zu detektierenden Unterzustand ein Sensor vorgesehen ist. Aufgrund der Ausgangsdaten des Sensors oder der Sensoren ist beispielsweise das Medizingerät nur einsetzbar, wenn das Trageteil fest mit mindestens einem der Supportteile verbunden ist. Ansonsten wird eine Benutzung des Medizingerätes unterbunden. Eine weitere Möglichkeit ist, dass die zweite Supportstruktur nur dann bewegbar ist, beispielsweise ein Stativ nur dann absenkbar (also in der Vertikalen verstellbar) ist, wenn eine feste Verbindung zwischen dem Trageteil und dem ersten Supportteil besteht. Ein Sensor ist beispielsweise ein optischer, mechanischer oder elektromechanischer Schalter. Ein Sensor zur Detektion einer Berührung zweier Teile erfasst zum Beispiel einen elektrischen Kontakt zwischen den beiden Teilen, beispielsweise durch Messung des Potentials an dem einen Teil, während an das andere Teil ein Prüfpotential angelegt wird.

In einer Ausgestaltungsform der Erfindung ist an zumindest einem der Teile des Adapters eine Markervorrichtung angeordnet. Eine Markervorrichtung ist eine Vorrichtung, deren Position und/oder Ausrichtung im Raum automatisch bestimmt werden kann, beispielsweise mittels einer Detektionsvorrichtung wie einer 3D-Kamera. Die Detektionsvorrichtung ist beispielsweise Bestandteil eines Navigationssystems, das zum Beispiel bei der bildgestützten Navigation (IGS, "Image Guided Surgery") Anwendung findet. Eine Markervorrichtung besteht beispielsweise aus drei Kugeln in bekannter räumlicher Anordnung. Aus der Lage einer Markervorrichtung kann auf die Lage des zugehörigen Teils des Adapters und damit auf die Lage des Medizingerätes oder einer Supportstruktur geschlossen werden, beispielsweise absolut oder bezogen auf eine (andere) Supportstruktur oder einer anatomischen Struktur, die mithilfe des Medizingeräts behandelt werden kann. Diese Information kann beispielsweise für die Navigation des mit dem Adapter verbundenen Medizingerätes oder eines anderen Medizingerätes verwendet werden.

Die vorliegende Erfindung soll anhand eines Ausführungsbeispiels näher erläutert werden. Dabei zeigt:
- Figur 1a - 1c: ein medizinisches System in drei Zuständen,
- Figur 2: einen dreiteiligen Adapter,
- Figur 3: das Trageteil des Adapters aus Figur 2,
- Figur 4: eine Explosionsdarstellung des hülsenförmigen Teils des Trageteils,
- Figur 5a - 5c: drei Ansichten des ersten Supportteils des Adapters aus Figur 2, und
- Figur 6: das zweite Supportteil des Adapters aus Figur 2.

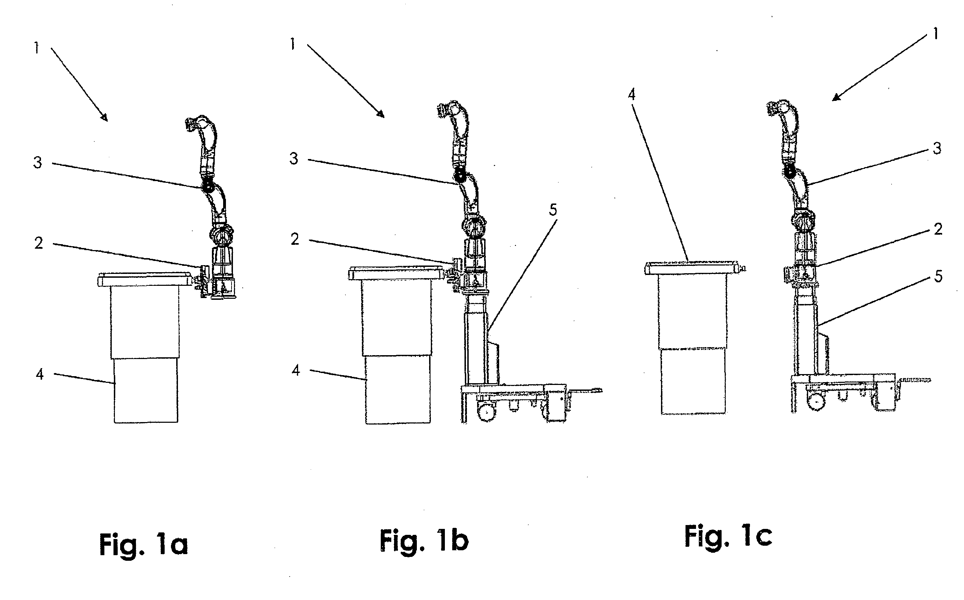

Dargestellt in den Figuren 1a bis 1c ist ein medizinisches System 1 mit einem Medizingerät in Form eines Roboterarms 3, einem Operationstisch 4, einem Stativ 5 und einem Adapter 2. Der Operationstisch 4 und das horizontal verfahrebare, vertikal höhenverstellbare, auf seine Füße absenkbare Stativ 5 stellen Supportstrukturen für den Roboterarm 3 dar. Mittels des Adapters 2 ist der Roboterarm 3 nur am Operationstisch 4 (siehe Figur 1a), sowohl am Operationstisch 4 als auch am Stativ 5 (siehe Figur 1b) oder nur am Stativ (siehe Figur 1c) befestigbar.

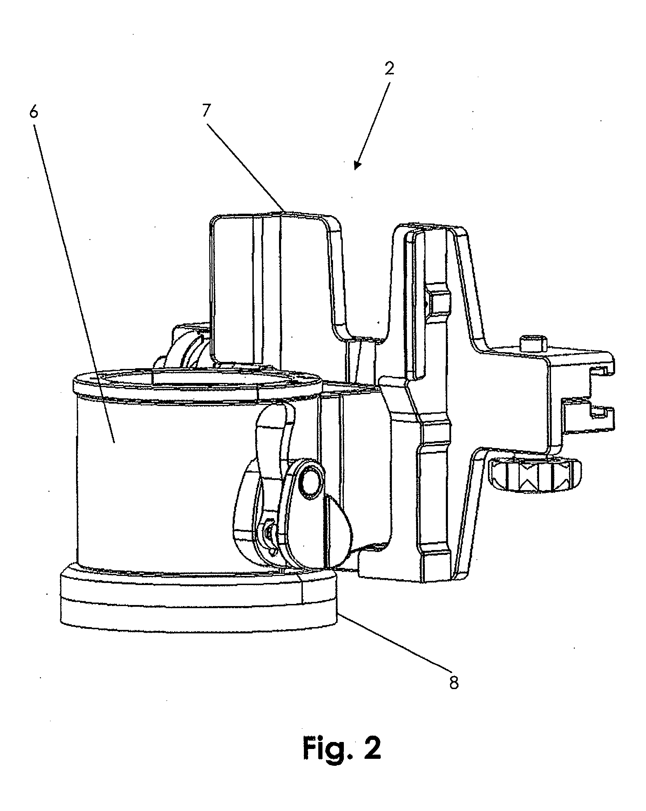

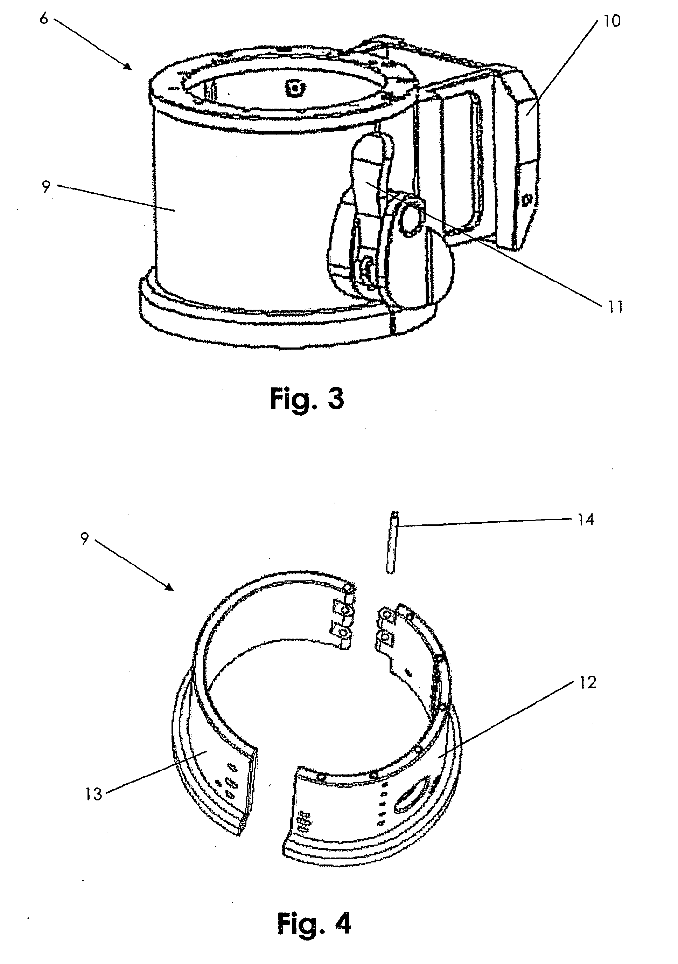

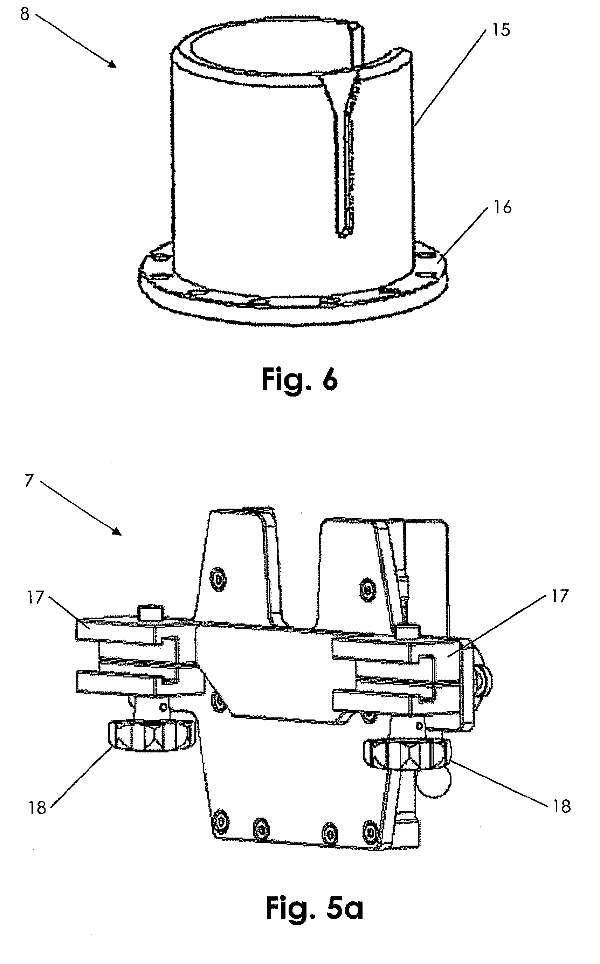

Der Adapter 2 ist detaillierter in Figur 2 dargestellt. Er besteht aus einem Trageteil 6 (siehe auch Figur 3), das fest mit dem Roboterarm 3 verbunden ist. Ein erstes Supportteil 7 des Adapters 2 (siehe Figuren 5a bis 5c) ist am Operationstisch 4 befestigt, ein zweites Supportteil 8 (siehe Figur 6) ist fest am Stativ 5 befestigt.

Die Figur 1a zeigt einen ersten Zustand des medizinischen Systems 1 und damit des Adapters 2, in dem der Roboterarm 3 ausschließlich am Operationstisch 4 befestigt ist. Dadurch sind der Operationstisch 4 und ein darauf befindlicher Patient für einen Operateur optimal zugänglich. In diesem Zustand ist das Trageteil 6 des Adapters 2 spielfrei nur mit dem ersten Supportteil 7 verbunden.

In dem in Figur 1b dargestellten Zustand wurde das Stativ 5 unter das Trageteil 6 gefahren, ausgefahren und eine Verbindung zwischen dem Trageteil 6 und dem zweiten Supportteil 8 hergestellt. Demnach ist das Trageteil 6 des Adapters 2 sowohl mit dem ersten Supportteil 7 als auch mit dem zweiten Supportteil 8 jeweils spielfrei verbunden. Das Gewicht des Roboterarm 3 wird somit sowohl vom Operationstisch 4 als auch vom Stativ 5 getragen, so dass die statischen und dynamischen Kräfte, die durch das Eigengewicht und eine Bewegung des Roboterarms 3 entstehen, von zwei Supportstrukturen getragen werden und so eine Bewegung des Operationstischs 4 verringert oder vermieden wird. Das Stativ 5 ist beispielsweise absenkbar, so dass es anstatt auf Rädern auf einem Basisteil oder Fuß ruht und somit eine noch höhere Standfestigkeit aufweist.

Soll der Roboterarm 3 vom Operationstisch 4 entfernt werden, so wird die Verbindung zwischen dem Trageteil 6 des Adapters 2 und dem ersten Supportteil 7 gelöst. Wird danach das Stativ ausgefahren oder die Absenkung auf das Basisteil aufgehoben, so wird der Roboterarm 3, wie in Figur 1c gezeigt, ausschließlich vom Stativ 5 getragen.

Die Figur 3 zeigt eine dreidimensionale Ansicht des Trageteils 6. Das Trageteil 6 besteht im Wesentlichen aus einem hülsenartigen Bereich 9 und einer Platte 10. Der hülsenartige Bereich 9 dient der spielfreien Fixierung mit dem zweiten Supportteil 8, die Platte 10 der spielfreien Verbindung mit dem ersten Supportteil 7.

Der hülsenartige Bereich 9 besteht, wie in Figur 4 dargestellt, im Wesentlichen aus zwei Halbschalen 12 und 13, die an einer ihrer Verbindungslinien mittels eines Stifts 14 scharnierartig verbunden sind. Die beiden Halbschalen 12 und 13 können somit einen zylinderartigen Innenraum bilden. An der anderen Verbindungslinie zwischen den beiden Halbschalen 12 und 13 ist der in Figur 3 dargestellte Exzenterhebel 11 vorgesehen, um die beiden Halbschalen 12 und 13 zu schließen, also aufeinander zuzubewegen. Werden die beiden Halbschalen 12 und 13 geschlossen, so wird eine Klemmkraft auf ein zylindrisches Grundteil 15 des zweiten Supportteils 8 (siehe Figur 6) zwischen den Halbschalen 12 und 13 ausgeübt, so dass eine spielfreie Fixierung zwischen dem Trageteil 6 und dem zweiten Supportteil 8 besteht. Das zweite Supportteil 8 weist darüber hinaus einen Flansch 16 auf, mittels dessen es auf dem Stativ 5 verschraubt ist.

Die äußere Kante des in den hülsenartigen Bereich 9 des Trageteils 6 einführbaren Grundteils 15 ist angeschrägt und bildet so eine Zentrierhilfe, die das Einführen des zweiten Supportteils 8 in den hülsenartigen Bereich des Trageteils 6 vereinfacht.

Aufgrund des zylindrischen Presssitzes des zweiten Supportteils 8 im Trageteil 6 ist eine spielfreie Fixierung der beiden Teile zueinander in verschiedenen Relaüvpositionen möglich. Dadurch kann beispielsweise ein Höhenausgleich geschaffen werden.

Die Figuren 5a bis 5c zeigen drei verschiedene dreidimensionale Ansichten des ersten Supportteils 7. In der Rückansicht in Figur 5a sind zwei C-förmige Klemmen 17 erkennbar, mittels derer das zweite Supportteil 7 an einer am Operationstisch 4 angeordneten Schiene befestigt wird. Über Rändelschrauben 18 wird eine Klemmkraft aufgebracht, die das erste Supportteil 7 sicher an der Schiene und damit am Operationstisch 4 fixiert.

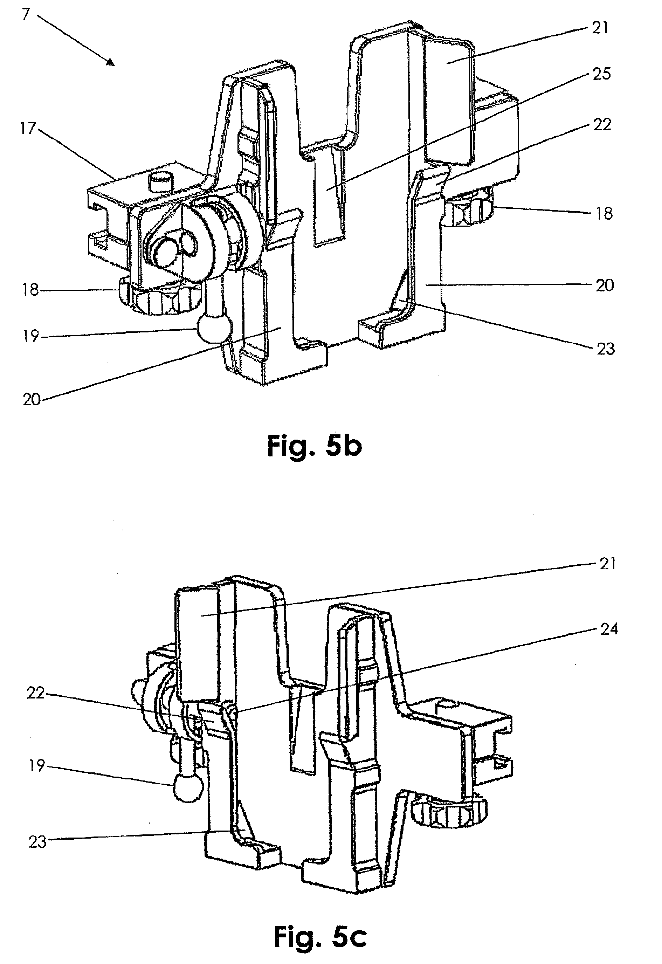

Die Figuren 5b und 5c zeigen die Vorderseite des ersten Supportteils 7 aus unterschiedlichen Blickrichtungen. Auf der Vorderseite einer Grundplatte des ersten Supportteils 7 sind zwei taschenartige Schienen 20 ausgebildet, in die die Platte 10 des Trageteils 6 einführbar ist. Um das Einführen der Platte 10 in die Schienen 20 zu vereinfachen, ist eine Zentrierhilfe mit zwei schrägen Leitflächen 21 und zwei weiteren schrägen Leitflächen 22 vorgesehen. Beim Annähern des Trageteils 6 an das erste Supportteil 7 erfolgt eine Führung der Platte 10 durch die sich trichterförmig erweiternden Führungsflächen 21, wodurch eine horizontale Zentrierung senkrecht zur Annäherungsrichtung erzielt wird. Beim Absenken der Platte 10 in die aus den Schienen 20 gebildete Tasche besorgen die beiden schrägen Führungsflächen 22 eine horizontale Zentrierung in der vorhergehend genannten Annäherungsrichtung.

Um einen spielfreien Sitz der Platte 10 in der Tasche zu gewährleisten, sind am Boden der Tasche zwischen der Grundplatte, dem Taschenboden und den Seiten der Schienen 20 zwei schräge Führungsflächen 23 vorgesehen. An diesen gleitet die Platte 10 ab, bis sie aufgrund der Schwerkraft spielfrei in den Schienen 20 sitzt. Durch die schrägen Führungsflächen 23 lassen sich Fertigungstoleranzen in der Platte 10 oder den Schienen 20 ausgleichen.

Die spielfreie Verbindung zwischen dem ersten Supportteil 7 und dem Trageteil 6 wird durch das Eigengewicht des Trageteils 6 und insbesondere des damit verbundenen Roboterarms 3 gewährleistet. Durch die Gewichtskraft wird die Platte 10 auf den Boden der durch die Schienen 20 gebildeten Tasche gedrückt, bis die Platte 10 über die Führungsflächen 23 in ihre Endposition rutscht. Somit ist die Platte 10 in horizontaler Richtung sowie in Richtung der Schwerkraft gegenüber dem ersten Supportteil 7 fixiert. Aufgrund des Eigengewichts des Trageteils 6 und des Roboterarms 3 ist ein Herausheben der Platte 10 aus dem taschenartigen Schienen 20 ohne willentliche äußere Krafteinwirkung nicht möglich.

Befindet sich die Platte 10 in ihrer Endposition am Boden der von den Schienen 20 gebildeten Tasche, so wird die Platte 10 und somit das Trageteil 6 durch Umlegen des Hebels 19 zusätzlich gesichert. Durch eine Bewegung des Hebels 19 wird ein Bolzen 24 in eine Arretierposition gebracht, in der er das Entfernen der Platte 10 aus den Schienen 20 verhindert. Optional erfüllt der Bolzen 24 nicht nur eine Sicherungsfunktion, sondern trägt auch zur spielfreien Fixierung von Trageteil 6 und erstem Supportteil 7 bei, indem er die Bewegungsfreiheit der Platte 10 nach oben verhindert. Alternativ erfolgt die spielfreie Fixierung ausschließlich durch den Bolzen 24.

Die Schienen 20 bilden eine Führungsvorrichtung zwischen dem ersten Supportteil 7 und dem Trageteil 6, der hülsenartige Bereich 9 bildet eine Führungsvorrichtung zwischen dem zweiten Supportteil 8 und dem Trageteil 6. Die Führungsvorrichtungen, die Platte 10 am Trageteil 6 und das zylinderartige Grundteil 15 am zweiten Supportteil 8 sind so ausgestaltet und angeordnet, dass die Vorschubrichtung der Platte 10 in den Schienen 20 und die Vorschubrichtung des Grundteils 15 im hülsenartigen Bereich 9 zueinander parallel sind. Bevorzugt sind diese Vorschubrichtungen vertikal, also in Richtung der Schwerkraft, orientiert.

Das medizinische System 1 weist vier optionale Sensoren auf. Der erste Sensor überwacht, ob ein Kontakt zwischen dem Trageteil 6 und dem ersten Supportteil 7 besteht. Der zweite Sensor überwacht, ob sich der Hebel 19 in einer Position befindet, in der der Bolzen 24 ein Herausrutschen der Platte 10 aus den taschenartigen Schienen 20 verhindert. Der dritte Sensor überwacht, ob ein Kontakt zwischen dem Trageteil 6 und dem zweiten Supportteil 8 besteht. Der vierte Sensor überwacht, ob sich der Hebel 11 in einer Position befindet, in der der hülsenartige Bereich 9 eine Klemmkraft auf das Grundteil 15 des zweiten Supportteils 8 ausübt.

Eine nicht dargestellte Steuerelektronik verhindert eine Betätigung des Roboterarms 3, wenn dieser nicht an mindestens einer Supportstruktur, also dem Operationstisch 4 oder dem Stativ 5, befestigt ist. Der Roboterarm 3 ist am Operationstisch 4 befestigt, wenn der erste Sensor einen Kontakt zwischen Trageteil 6 und erstem Supportteil 7 detektiert und der zweite Sensor einen geschlossenen Bolzen 24 detektiert. Eine feste Verbindung zwischen dem Roboterarm 3 und dem Stativ 5 besteht, wenn der dritte Sensor einen Kontakt zwischen dem Trageteil 6 und dem zweiten Supportteil 8 detektiert und der vierte Sensor den geschlossenen Zustand des Hebels 11 detektiert. Die Ausgangsdaten der vier Sensoren können weiterhin dazu verwendet werden, eine Höhenverstellung des Stativs 5 nur dann zuzulassen, wenn das Trageteil 6 gegenüber dem ersten Supportteil 7 fixiert ist.

In den Figuren nicht erkennbar ist eine Sicherungsvorrichtung, die eine Relativbewegung des zylindrischen Grundteils 15 des zweiten Supportteils 8 im hülsenartigen Bereich 9 des Trageteils 6 verhindert, solange sich die Platte 10 des Trageteils 6 nicht in ihrer Endposition in der aus den Schienen 20 gebildeten Tasche am ersten Supportteil 7 befindet. Dazu wird beispielsweise ein Bolzen verwendet, der in einer Position die vorgenannte Relativbewegung blockiert. Dieser Bolzen wird über einen Hebelmechanismus radial von der Mittelachse des hülsenartigen Bereichs 9 wegbewegt, wenn der Hebel über die schräge Fläche 25 in der Grundplatte des ersten Supportteils 7 gleitet. Dies ist der Fall, wenn die Platte 10 des Trageteils 6 in die aus den Schienen 20 gebildete Tasche am ersten Supportteil 7 geschoben wird.

## Patentansprüche

1. Adapter (2) zur Fixierung eines Medizingerätes (3) an einer oder zwei Supportstrukturen (4, 5), wobei der Adapter (2) dreiteilig aus einem Trageteil (6) und zwei Supportteilen (7, 8) aufgebaut ist, wobei das Trageteil (6) mit dem Medizingerät (3) verbindbar ist, das erste Supportteil (7) mit einer ersten Supportstruktur (4) verbindbar ist und das zweite Supportteil (8) mit einer zweiten Supportstruktur (5) verbindbar ist, und der Adapter (2) zumindest drei Zustände einnehmen kann, wobei im ersten Zustand das Trageteil (6) spielfrei nur mit dem ersten Supportteil (7) verbunden ist, im zweiten Zustand das Trageteil (6) spielfrei nur mit dem zweiten Supportteil (8) verbunden ist und im dritten Zustand das Trageteil (6) spielfrei mit dem ersten Supportteil (7) und dem zweiten Supportteil (8) verbunden ist, **gekennzeichnet durch** ein Verschiebeteil (10) am Trageteil (6), das dazu ausgebildet ist, an einem Element des ersten Supportteils (7) verschiebbar angebracht zu werden.

2. Adapter (2) nach Anspruch 1, **dadurch gekennzeichnet, dass** das Trageteil (6) und das erste Supportteil (7) in genau einer Position spielfrei miteinander verbindbar sind.

3. Adapter (2) nach einem der Ansprüche 1 oder 2, **dadurch gekennzeichnet, dass** das Verschiebeteil (10) dazu ausgebildet ist, in taschenartige Schienen (20) am ersten Supportteil (7) eingeführt zu werden.

4. Adapter (2) nach Anspruch 3, **gekennzeichnet durch** mindestens eine schräge Anlagefläche (23) am Ende der taschenartigen Schienen (20) am ersten Supportteil (7).

5. Adapter (2) nach einem der Ansprüche 3 oder 4, **gekennzeichnet durch** eine Zentrierhilfe (21, 22) zur Vereinfachung der Verbindung zwischen dem Trageteil (6) und dem ersten Supportteil (7).

6. Adapter (2) nach einem der Ansprüche 1 bis 5, **gekennzeichnet durch** einen Verschluss (19, 24) zur Sicherung des Trageteils (6) gegenüber dem ersten Supportteil (7).

7. Adapter (2) nach einem der Ansprüche 1 bis 6, **dadurch gekennzeichnet, dass** das Trageteil (6) und das zweite Supportteil (8) in mehreren Relativpositionen spielfrei miteinander verbindbar sind.

8. Adapter (2) nach einem der Ansprüche 1 bis 7, **dadurch gekennzeichnet, dass** das zweite Supportteil (8) ein zylindrisches Grundteil (15) aufweist, das in einen hülsenartigen Bereich (9) des Trageteils (6) einführbar ist.

9. Adapter (2) nach Anspruch 8, **dadurch gekennzeichnet, dass** der hülsenartige Bereich (9) des Trageteils (6) aus zwei Halbschalen (12, 13) besteht, die mittels eines Scharniers miteinander verbunden sind und durch einen Verschlussmechanismus (11) verschließbar sind.

10. Adapter (2) nach einem der Ansprüche 1 bis 9, **gekennzeichnet durch** eine Zentrierhilfe zur Vereinfachung der Verbindung zwischen dem Trageteil (6) und dem zweiten Supportteil (8).

11. Adapter (2) nach einem der Ansprüche 1 bis 10, **dadurch gekennzeichnet, dass** die Verbindung zwischen dem Trageteil (6) und dem zweiten Supportteil (8) erst mittels eines Hebelmechanismus trennbar ist, wenn eine Verbindung zwischen dem Trageteil (6) und dem ersten Supportteil (7) besteht.

12. Medizinisches System (1), aufweisend ein Medizingerät (3), eine erste Supportstruktur (4), eine zweite Supportstruktur (5) und einen Adapter (2) nach einem der Ansprüche 1 bis 11.

13. Medizinisches System (1) nach Anspruch 12, **gekennzeichnet durch** mindestens einen Sensor zur Erfassung eines Zustands des Adapters (2).

14. Medizinisches System (1) nach einem der Ansprüche 12 oder 13, **gekennzeichnet durch** eine Markervorrichtung an zumindest einem der Teile (6, 7, 8) des Adapters (2).

## Claims

1. An adaptor (2) for fixedly attaching a medical device (3) to one or two support structures (4, 5), wherein the adaptor (2) is a three-part adaptor comprising a carrying part (6) and two support parts (7, 8), wherein the carrying part (6) is connectable to the medical device (3), the first support part (7) is connectable to a first support structure (4) and the second support part (8) is connectable to a second support structure (5) and the adaptor (2) can assume at least three states, wherein the carrying part (6) is, in the first state, connected free of play only with the first support part (7), the carrying part (6) is, in the second state, connected free of play only with the second support part (8) and the carrying part (6) is, in the third state, connected free of play with the first support part (7) and the second support part (8), **characterized by** a slide part (10) on the carrying part (6) which is adapted to be slidably attached to an element of the first support part (7).

2. The adaptor (2) of claim 1, **characterized in that** the carrying part (6) and the first support part (7) are connectable with each other free of play in exactly one position.

3. The adaptor (2) of one of claims 1 or 2, **characterized in that** the slide part (10) is designed such that it can be inserted into pocket-like rails (20) at the first support part (7).

4. The adaptor (2) of claim 3, **characterized by** at least one slanted stop face (23) at the end of the pocket-like rails (20) at the first support part (7).

5. The adaptor (2) of one of claims 3 or 4, **characterized by** a centering aid (21, 22) for simplifying the connection between the carrying part (6) and the first support part (7).

6. The adaptor (2) of one of claims 1 to 5, **characterized by** a lock (19, 24) for securing the carrying part (6) versus the first support part (7).

7. The adaptor (2) of one of claims 1 to 6, **characterized in that** the carrying part (6) and the second support part (8) can be connected with each other free of play in a plurality of relative positions.

8. The adaptor (2) of one of claims 1 to 7, **characterized in that** the second support part (8) comprises a cylindrical base part (15) which is insertable into a sleeve-like area (9) of the carrying part (6).

9. The adaptor (2) of claim 8, **characterized in that** the sleeve-like area (9) of the carrying part (6) consists of two half-shells (12, 13) which are connected via a hinge and which are closable via a lock mechanism (11).

10. The adaptor (2) of one of claims 1 to 9, **characterized by** centering eight for simplifying the connection between the carrying part (6) and the second support part (8).

11. The adaptor (2) of one of claims 1 to 10, **characterized in that** the connection between the carrying part (6) and the second support part (8) is only releasable via a lever mechanism when there is a connection between the carrying part (6) and the first support part (7).

12. A medical system (1) comprising a medical device (3), a first support structure (4), a second support structure (5) and an adaptor (2) according to one of claims 1 to 11.

13. The medical system (1) of claim 12, **characterized by** at least one sensor for detecting the state of the adaptor (2).

14. The medical system (1) of one of claims 12 or 13, **characterized by** a marker device on at least one of the parts (6, 7, 8) of the adaptor (2).

## Revendications

1. Adaptateur (2) pour la fixation d'un dispositif médical (3) à une ou deux structures de support (4, 5), où l'adaptateur (2) est composé de trois parties, une partie porteuse (6) et deux parties de support (7, 8), où la partie porteuse (6) peut être connectée au dispositif médical (3), la première partie de support (7) peut être connectée à une première structure de support (4) et la deuxième partie de support (7) peut être connectée à une deuxième structure de support (5), et l'adaptateur (2) peut se trouver dans trois états, où, dans le premier état, la partie porteuse (6) est connectée sans jeu uniquement avec la première partie de support (7), dans le deuxième état, la partie porteuse (6) est connectée sans jeu uniquement avec la deuxième partie de support (8) et, dans le troisième état, la partie porteuse (6) est connectée sans jeu avec la première partie de support (7) et la deuxième partie de support (8), **caractérisé par** une pièce coulissante (10) sur la partie porteuse (6) conçue pour être attachée de manière coulissante à un élément de la première partie de support (7).

2. Adaptateur (2) selon la revendication 1, **caractérisé en ce que** la partie porteuse (6) et la première partie de support (7) peuvent être connectées l'une à l'autre sans jeu dans une position précise.

3. Adaptateur (2) selon l'une quelconque des revendications 1 ou 2, **caractérisé en ce que** la pièce coulissante (10) est conçue pour être introduite dans des rails (20) en forme de poche (20) situés sur la première partie de support (7).

4. Adaptateur (2) selon la revendication 3, **caractérisé par** au moins une surface d'appui (23) oblique à l'extrémité des rails (20) en forme de poche situés sur la première partie de support (7).

5. Adaptateur (2) selon l'une quelconque des revendications 3 ou 4, **caractérisé par** un dispositif de centrage (21, 22) pour simplifier la connexion entre la partie porteuse (6) et la première partie de support (7).

6. Adaptateur (2) selon l'une quelconque des revendications 1 à 5, **caractérisé par** une fermeture (19, 24) pour la sécurisation de la partie porteuse (6) par rapport à la première partie de support (7).

7. Adaptateur (2) selon l'une quelconque des revendications 1 à 6, **caractérisé en ce que** la partie porteuse (6) et la deuxième partie de support (8) peuvent être connectées l'une à l'autre sans jeu dans plusieurs positions relatives.

8. Adaptateur (2) selon l'une quelconque des revendications 1 à 7, **caractérisé en ce que** la deuxième partie de support (8) comporte une partie de base (15) cylindrique pouvant être introduite dans une région en forme de manchon (9) de la partie porteuse (6).

9. Adaptateur selon la revendication 8, **caractérisé en ce que** la région en forme de manchon (9) de la partie porteuse (6) est composée de deux demi-coques (12, 13) connectées au moyen d'une charnière et pouvant être fermées par un mécanisme de fermeture (11).

10. Adaptateur selon l'une quelconque des revendications 1 à 9, **caractérisé par** un dispositif de centrage pour simplifier la connexion entre la partie porteuse (6) et la deuxième partie de support (8).

11. Adaptateur selon l'une quelconque des revendications 1 à 10, **caractérisé en ce que** la déconnexion entre la partie porteuse (6) et la deuxième partie de support (8) ne peut être effectuée au moyen d'un mécanisme de levier que lorsqu'il y a une connexion entre la partie porteuse (6) et la première partie de support (7).

12. Système médical (1) comportant un dispositif médical (3), une première structure de support (4), une deuxième structure de support (5) et un adaptateur (2) selon l'une quelconque des revendications 1 à 11.

13. Système médical (1) selon la revendication 12, **caractérisé par** au moins un détecteur destiné à détecter un état de l'adaptateur (2).

14. Système médical (1) selon l'une quelconque des revendications 12 ou 13, **caractérisé par** un dispositif de marqueur sur au moins une partie (6, 7, 8) de l'adaptateur (2).
